# FASCICULE DE BREVET EUROPEEN

(11) **EP 1 048 637 B1**
(45) Date de publication et mention de la délivrance du brevet: **29.01.2003**
(21) Numéro de dépôt: 00401126.8
(22) Date de dépôt: 21.04.2000
(51) Int. Cl.: C07C 29/141, C07C 29/145

(54) **Procédé de préparation d'un mélange de mannitol et de sorbitol par hydrogénation continue de la glucosone**
Verfahren zur Herstellung einer Mischung von Mannit und Sorbit durch kontinuierliche Hydrierung von Glucoson
Process for the preparation of a mixture of mannitol and sorbitol by continuous hydrogenation of glucosone

(30) Priorité: 27.04.1999 FR 9905308
(43) Date de publication de la demande: 02.11.2000
(73) Titulaire: Roquette Frères, 62136 Lestrem (FR)
(72) Inventeur: Choque, Jean-Christphe, 59000 Lille (FR); Fleche, Guy, 59190 Hazebrouck (FR)
(74) Mandataire: Boulinguiez, Didier

(56) Documents cités:
- WO-A-84/00778

## Description

La présente invention est relative à un procédé de préparation d'un mélange de mannitol et de sorbitol par hydrogénation de la glucosone.

La présente invention est surtout relative à un procédé de préparation d'un mélange de mannitol et de sorbitol contenant une proportion élevée en mannitol, obtenu en conduisant l'hydrogénation de la glucosone en mode continu.

La présente invention concerne également un procédé continu de préparation dudit mélange de mannitol et de sorbitol, par hydrogénation continue de la glucosone en lit fixe de catalyseur, et plus particulièrement la mise en oeuvre de ce. procédé continu dans une succession de lits fixes de catalyseur disposés en série et en deux zones de réaction.

La préparation industrielle du mannitol et du sorbitol est classiquement effectuée par hydrogénation catalytique du glucose, du fructose, ou d'un mélange de ces deux sucres.

Ledit mélange de glucose et de fructose est obtenu classiquement à partir du sucre inverti (glucose et fructose en proportion 50/50).

Cependant, il est communément admis que la réduction catalytique du sucre inverti, par exemple au nickel de Raney, conduit à la fabrication d'un mélange de mannitol et de sorbitol à faible proportion en mannitol, puisque l'on obtient une mole de mannitol pour trois moles de sorbitol.

En effet, l'hydrogénation catalytique du fructose pur conduit déjà à un mélange équimolaire de mannitol et de sorbitol, tandis que l'hydrogénation catalytique du glucose donne directement du sorbitol.

Tous les procédés habituellement mis en oeuvre par hydrogénation catalytique du fructose, ou du mélange du fructose et du glucose, ne permettent donc pas de produire plus de 50 % de mannitol.

Pour résoudre cette difficulté, le brevet WO 84.00778 décrit un procédé de préparation d'un mélange de mannitol et de sorbitol, dans lequel le mannitol représente au moins 60 % du mélange, par l'hydrogénation de la glucosone à l'aide d'un catalyseur au nickel de Raney.

La glucosone (également connue sous les noms de 2 céto-glucose ou de D-arabino-2-hexulosone) est classiquement obtenue par oxydation du glucose par voie chimique (par traitement à l'eau oxygénée ou avec de l'acétate de cuivre) ou avantageusement par voie enzymatique (à l'aide d'une pyranose 2-oxidase) afin de conduire à un produit de haute pureté

Il est décrit dans ce brevet que la conduite de l'hydrogénation en mode discontinu de la glucosone, quelle qu'en soit la matière sèche mise en oeuvre, avec un catalyseur au nickel de Raney, permet de préparer des mélanges de mannitol et de sorbitol contenant des proportions variables en mannitol, comprises entre 60 et 75 %.

Cependant, les conditions d'hydrogénation mises en oeuvre dans cette demande de brevet conduisent à produire tout d'abord un mélange de mannose, fructose et glucose à partir de la glucosone, avant que la poursuite de l'hydrogénation ne permette d'obtenir le mélange de mannitol et de sorbitol. Les temps de réaction pour hydrogéner la glucosone à haute matière sèche sont très longs et donc pénalisants.

De plus, même si ce procédé d'hydrogénation discontinu permet d'obtenir le mélange de mannitol et de sorbitol, à partir d'une solution de glucosone à 30 % de matière sèche, avec une proportion de mannitol dans ledit mélange de l'ordre de 70 %, ce n'est qu'avec une sélectivité de l'ordre de 97 %, et une médiocre productivité. De plus, le taux de conversion n'est que de 90 %.

Il apparaît donc clairement dans l'état de la technique que les procédés d'hydrogénation en mode discontinu de la glucosone à haute matière sèche ne permettent pas d'obtenir un mélange de mannitol et de sorbitol contenant une proportion élevée en mannitol, avec un taux de conversion, une sélectivité et une productivité élevées.

L'invention a donc pour but de remédier à cette situation, et de proposer un moyen permettant d'atteindre un meilleur compromis entre taux de conversion, sélectivité et productivité, et ce pour une matière sèche élevée en glucosone.

La société Demanderesse a trouvé, après de nombreuses recherches, qu'un tel moyen pouvait consister en un procédé continu d'hydrogénation catalytique de la glucosone à haute matière sèche, conduite dans des conditions particulières.

La présente invention a précisément pour objet un procédé de préparation d'un mélange de mannitol et de sorbitol, caractérisé par le fait que l'on conduit l'hydrogénation continue de la glucosone d'une matière sèche au moins égale à 10 %, de préférence comprise entre 30 et 50 %, dans une succession de lits fixes de catalyseur disposés en série qui comprend :
- une première zone d'hydrogénation, constituée d'au moins un lit fixe de catalyseur, où on conduit l'hydrogénation à une température au plus égale à 80°C, de préférence comprise entre 50 et 80°C,
- une seconde zone d'hydrogénation, constituée d'au moins un lit fixe de catalyseur, où on conduit l'hydrogénation à une température au moins égale à 80°C, de préférence comprise entre 100 et 150°C.

On choisit de préparer la solution à hydrogéner avec de la glucosone de très haute pureté, avantageusement produite par voie enzymatique à partir du glucose par tout moyen connu de l'homme du métier.

On entend par « haute pureté », une teneur en glucosone de l'ordre de 100 %.

Le catalyseur est choisi dans le groupe constitué du palladium, du nickel, du ruthénium, du platine, du rhodium, du cobalt, du cuivre, du zinc, du chrome, du manganèse, du tungstène, et est de préférence du ruthénium.

Dans le procédé conforme à l'invention, le catalyseur est généralement imprégné ou co-échangé sur un support inerte, de préférence choisi dans le groupe constitué du charbon actif, de la tourbe, des zéolites, des aluminosilicates, du dioxyde de titane. De préférence il est constitué de charbon actif.

Le rapport pondéral catalyseur / support inerte est établi avantageusement à une valeur comprise entre 1 et 5 %, de préférence de l'ordre de 2 % comme exemplifié ci-après.

Il est également possible de mettre en oeuvre un catalyseur comportant un agent promoteur. Cet agent promoteur peut être choisi dans le groupe constitué du titane, du molybdène et du platine.

Le catalyseur est disposé dans un lit fixe sous la forme d'un empilement compact de particules, le tout placé sur des grilles de soutien dans un réacteur d'hydrogénation. On choisit avantageusement un réacteur à ruissellement.

Au sens de l'invention, on entend par « réacteur à ruissellement », un réacteur d'hydrogénation dans lequel une phase liquide contenant le produit à hydrogéner et une phase gaz circulent de manière co-courante ou contre-courante, de préférence de manière co-courante de haut en bas, dans un lit fixe de particules de catalyseur où s'effectue la réaction d'hydrogénation.

Les débits d'écoulement de ces deux phases sont réglés pour permettre au liquide de ruisseler sur lesdites particules de catalyseur, et assurer le meilleur contact entre les deux phases liquide et gazeuse d'une part, et la phase solide du catalyseur d'autre part.

Dans un mode de réalisation du procédé conforme à l'invention, on choisit de préparer un lit fixe constitué de 200 l de catalyseur commercial, un débit d'alimentation de la solution de glucosone d'une matière sèche comprise entre 10 et 50 % en poids, à une valeur comprise entre 150 et 350 kg/h, et une quantité d'hydrogène introduite dans ledit réacteur à ruissellement de l'ordre de deux à quinze fois la stoechiométrie de la réaction.

Dans un mode de réalisation du procédé conforme à l'invention, on choisit de mettre en oeuvre un procédé continu d'hydrogénation de la glucosone dans une succession de lits fixes de catalyseur disposés en série et en au moins deux zones de réaction.

L'hydrogénation est alors avantageusement conduite dans une première zone de réaction, constituée d'au moins un lit fixe de catalyseur, de manière à obtenir une conversion totale de la glucosone en un mélange de mannose, glucose et fructose, contenant une proportion élevée en mannose, puis dans une seconde zone de réaction, constituée d'au moins un lit fixe de catalyseur, où l'hydrogénation est conduite de manière à obtenir un mélange de mannitol et de sorbitol avec un taux de conversion et une sélectivité élevés.

La société Demanderesse a ainsi trouvé qu'il est possible, contrairement à ce qui est établi dans l'état la technique, de conduire l'hydrogénation en mode continu et en deux zones de réaction, de manière à séparer l'étape de production du mélange de mannose, glucose et fructose de celle de production du mélange de mannitol et de sorbitol, pour obtenir ledit mélange de mannitol et de sorbitol avec une teneur élevée en mannitol encore jamais atteinte, et avec une productivité, une sélectivité et un taux de conversion élevés.

On peut mettre en oeuvre des catalyseurs de nature différente dans chacune des zones de réaction. Cependant on préfère utiliser un catalyseur de même nature dans ces deux zones.

Dans la première zone de réaction, les conditions de température sont réglées de manière à obtenir une conversion élevée, de l'ordre de 100 % de la glucosone en un mélange de mannose, glucose et fructose, dont la proportion en mannose est élevée.

Au sens de l'invention, on entend par « proportion en mannose élevée », une teneur en mannose au moins égale à 70 % en poids du mélange.

On met en oeuvre également des conditions de température qui limitent également, voire annulent la conversion de la glucosone en fructose, et limite celle du glucose.

La température d'hydrogénation dans la première zone de réaction est donc fixée à une valeur au plus égale à 80°c, de préférence comprise entre 50 et 80°C.

Dans la seconde zone de réaction, les conditions de température sont réglées de manière à obtenir le mélange de mannitol et de sorbitol, contenant le mannitol en proportion élevée, avec un taux de conversion au moins égal à 98 %.

Au sens de l'invention, on entend par « proportion élevée en mannitol », un rapport pondéral mannitol sur sorbitol au moins égal à 3,5.

La température d'hydrogénation dans la seconde zone de réaction est donc fixée à une valeur au moins égale à 80°c, de préférence comprise entre 100 et 150°C.

Dans le procédé conforme à l'invention, on choisit de conduire l'hydrogénation dans au moins un lit fixe de catalyseur dans lequel cette pression est supérieure à 50 bars.

De préférence, la pression d'hydrogénation est maintenue constante dans les deux zones de réaction à une valeur comprise entre au plus 50 bars et 150 bars, et est de préférence comprise entre 55 et 100 bars.

Les lits fixes de catalyseur sont avantageusement disposés dans des réacteurs à ruissellement. On choisit de mettre en oeuvre 200 l de particules de catalyseur commercial, un débit d'alimentation de la solution de glucosone d'une matière sèche comprise entre 10 et 50 % en poids à une valeur comprise entre 150 et 350 kg/h, et une quantité d'hydrogène comprise entre deux et quinze fois la stoechiométrie de la réaction.

D'autres caractéristiques et avantages de l'invention apparaîtront à la lecture de l'exemple non limitatif décrit ci-dessous.

### Exemple

La réaction d'hydrogénation est effectuée dans deux réacteurs à ruissellement connectés en série avec un échangeur thermique intermédiaire.

Les deux réacteurs renferment chacun un unique lit fixe de catalyseur au ruthénium sur charbon, dans lequel on fait circuler l'hydrogène et la solution de glucosone de manière co-courante du haut vers le bas desdits réacteurs.

Chaque lit fixe de catalyseur au ruthénium est constitué d'un empilement compact de grains de catalyseur cylindriques.

Chaque grain de catalyseur est composé de charbon actif de type NORIT RX08, sous la forme d'un cylindre de 0,8 mm de diamètre et de 1 à 5 mm de longueur, contenant 2 % en poids de ruthénium.

Chaque réacteur contient de l'ordre 200 1 de catalyseur, disposé en lit fixe de 30 cm de diamètre et de l'ordre de 3 m de hauteur.

On alimente simultanément la première zone d'hydrogénation avec une solution de glucosone à 30 % de matière sèche, à un débit de 200 kg/h et de l'hydrogène à raison de 10 kg/h.

La pression de fonctionnement dans le premier réacteur est de 55 bars, et la température est fixée à 80°C.

La température d'entrée du second réacteur est amenée à 100°C, et la pression maintenue à 55 bars.

A la sortie du premier réacteur, le taux de conversion est de 100 % et le mélange de mannose, fructose et glucose présente la composition suivante : 74 % en poids de mannose, 8% en poids de fructose, et 16 % en poids de glucose.

A la sortie du second réacteur, la conversion est de 99 %, et le mélange de mannitol sur sorbitol présente un rapport de mannitol sur sorbitol de 3,8.

## Revendications

1. Procédé de préparation d'un mélange de mannitol et de sorbitol, **caractérisé par le fait que** l'on conduit l'hydrogénation continue de la glucosone d'une matière sèche au moins égale à 10 %, de préférence comprise entre 30 et 50 %, dans une succession de lits fixes de catalyseur disposés en série qui comprend :
- une première zone d'hydrogénation, constituée d'au moins un lit fixe de catalyseur, où on conduit l'hydrogénation à une température au plus égale à 80°C, de préférence comprise entre 50 et 80°C,
- une seconde zone d'hydrogénation, constituée d'au moins un lit fixe de catalyseur, où on conduit l'hydrogénation à une température au moins égale à 80°C, de préférence comprise entre 100 et 150°C.

2. Procédé selon la revendication 1, **caractérisé par le fait que** le catalyseur est choisi dans le groupe constitué du palladium, du nickel, du ruthénium, du platine, du rhodium, du cobalt, du cuivre, du zinc, du chrome, du manganèse, du tungstène, et est de préférence du ruthénium.

3. Procédé selon l'une ou l'autre des revendications 1 et 2, **caractérisé par le fait que** l'hydrogénation est conduite dans au moins un lit fixe de catalyseur à une pression supérieure à 50 bars.

4. Procédé selon l'une quelconque des revendications 1 à 3, **caractérisé par le fait que** la pression d'hydrogénation dans les deux zones de réaction est comprise entre au plus 50 bars et 150 bars, et est de préférence comprise entre 55 et 100 bars.

## Patentansprüche

1. Verfahren zur Herstellung einer Mischung von Mannit und Sorbit, **dadurch gekennzeichnet, dass** man die kontinuierliche Hydrierung von Glucoson mit einer Trockenmasse von mindestens gleich 10%, vorzugsweise zwischen 30 und 50%, in einer Folge von Katalysator-Festbetten durchführt, die in Reihe angeordnet sind, die umfasst:
- eine erste Hydrierungszone, die aus mindestens einem Katalysator-Festbett besteht, in dem die Hydrierung bei einer Temperatur von höchstens gleich 80°C, vorzugsweise zwischen 50 und 80°C, durchgeführt wird,
- eine zweite Hydrierungszone, die aus mindestens einem Katalysator-Festbett besteht, in dem die Hydrierung bei einer Temperatur von mindestens gleich 80°C, vorzugsweise zwischen 100 und 150°C, durchgeführt wird.

2. Verfahren nach Anspruch 1, **dadurch gekennzeichnet, dass** der Katalysator aus der Gruppe ausgewählt ist, die aus Palladium, Nickel, Ruthenium, Platin, Rhodium, Cobalt, Kupfer, Zink, Chrom, Mangan, Wolfram besteht, und vorzugsweise Ruthenium ist.

3. Verfahren nach einem der Ansprüche 1 und 2, **dadurch gekennzeichnet, dass** die Hydrierung in mindestens einem Katalysator-Festbett bei einem Druck über 50 bar durchgeführt wird.

4. Verfahren nach einem der Ansprüche 1 bis 3, **dadurch gekennzeichnet, dass** der Hydrierungsdruck in den beiden Reaktionszonen höchstens zwischen 50 bar und 150 bar liegt und vorzugsweise zwischen 55 und 100 bar liegt.

## Claims

1. Method of preparing a mixture of mannitol and sorbitol, **characterised by** the fact that the continuous hydrogenation of glucosone with a dry matter equal to at least 10%, preferably between 30 and 50%, is carried out in a succession of fixed beds catalyst disposed in series, which comprises:
- a first hydrogenation zone, consisting of at least one fixed bed catalyst, where the hydrogenation is carried out at a temperature equal at the most to 80°C, preferably comprised between 50°C and 80°C,
- a second hydrogenation zone, consisting of at least one fixed bed catalyst, where the hydrogenation is carried out at a temperature equal to at least 80°C, preferably comprised between 100 and 150°C.

2. Method according to claim 1, **characterised by** the fact that the catalyst is chosen from the group consisting of palladium, nickel, ruthenium, platinium, rhodium, cobalt, copper, zinc, chromium, manganese, tungsten, and is preferably ruthenium.

3. Method according to one or other of claims 1 and 2, **characterised by** the fact that the hydrogenation is carried out in at least one fixed bed catalyst at a pressure greater than 50 bar.

4. Method according to any one of claims 1 to 3, **characterised by** the fact that the hydrogenation pressure in the two reaction zones is between at the most 50 bar and 150 bar, and preferably between 55 and 100 bar.
